# EUROPÄISCHE PATENTSCHRIFT

(11) **EP 4 562 397 B1**
(45) Veröffentlichungstag und Bekanntmachung des Hinweises auf die Patenterteilung: **31.12.2025**
(21) Anmeldenummer: 23758592.2
(22) Anmeldetag: 17.08.2023
(51) Int. Cl.: G01N 15/08, G01N 33/18, B01D 65/10, C02F 1/28

(54) **VERFAHREN ZUR BESTIMMUNG DER SÄTTIGUNGSDAUER EINES ADSORPTIONSMITTELS**
METHOD FOR DETERMINING THE SATURATION TIME OF AN ADSORBENT
PROCÉDÉ DE DÉTERMINATION LE TEMPS DE SATURATION D'UN ADSORBANT

(30) Priorität: 15.09.2022 DE 102022123613
(43) Veröffentlichungstag der Anmeldung: 04.06.2025
(73) Patentinhaber: CERAFILTEC Germany GmbH, 66121 Saarbrücken (DE)
(72) Erfinder: GABRIEL, Kay Gunther, Dubai (AE); KASCHEK, Martin, 66386 St. Ingbert (DE)
(74) Vertreter: Grättinger Möhring von Poschinger Patentanwälte Partnerschaft mbB
(86) Internationale Anmeldenummer: PCT/EP2023/072703
(87) Internationale Veröffentlichungsnummer: WO 2024/056312

(56) Entgegenhaltungen:
- EP-A1- 3 437 720
- WO-A1-2010/088720
- DE-A1- 19 962 791

## Beschreibung

Die vorliegende Erfindung betrifft ein Verfahren zur Bestimmung der Sättigungsdauer eines Adsorptionsmittels und ein Verfahren zur Abschätzung des DOC-Anteils eines zu filtrierenden Testmediums.

Der DOC-Anteil (DOC = Dissolved Organic Carbon) beschreibt den Anteil des gelösten organischen Kohlenstoffs in einem flüssigen Medium. Als ein sogenannter organischer Summenparameter ist der DOC-Anteil damit ein Maß für die Gesamtheit aller gelösten organischen Verbindungen und stellt einen wichtigen Parameter zur Charakterisierung der Wassergüte dar.

Der DOC-Anteil bildet zusammen mit dem partikulären (ungelösten) organischen Kohlenstoff (POC = Particular Organic Carbon) und dem flüchtigen organischen Kohlenstoff (VOC = volatile organic carbon) den gesamten organischen Gesamtkohlenstoff (TOC = total organic carbon). Die Abtrennung des partikulären organischen Kohlenstoffs erfolgt üblicherweise über einen groben Mikrofilter mit einem Porendurchmesser von etwa 0,45 µm.

Das direkte Messen von organischen Summenparametern stellt in der Praxis eine Herausforderung dar. So muss bspw. beim Summenparameter Biologischer Sauerstoffbedarf (BSB) eine Wasserprobe über ein mehrtägiges Testverfahren gemessen werden. Beim Chemischen Sauerstoffbedarf (CSB) sind in der Regel auch mehrere Stunden notwendig, bis das Messergebnis feststeht. Es ist damit nicht möglich, direkt auf kurzfristige Änderungen der Konzentration organischer Inhaltsstoffe im Wasser betriebstechnisch zu reagieren.

Ähnlich verhält es sich mit der Messung des TOC-Anteils und des DOC-Anteils. Die verschiedenen Messmethoden werden bspw. in der Normung DIN EN1484, ASTM D4839, ASTM D4779 beschrieben.

Gemäß einer ersten im Stand der Technik bekannten Messmethode wird der TOC bzw. der DOC-Anteil ermittelt, indem mittels eines Verbrennungsverfahren eine Probe der zu bestimmenden Flüssigkeit unter Zugabe von Sauerstoff üblicherweise auf ca. 700 bis 1.000 °C erhitzt wird, sodass sich der enthaltene organische Kohlenstoff vollständig zu Kohlenstoffdioxid (CO2) umwandelt. Die Bestimmung der der Masse des CO2 wird anschließend über sogenannte nicht dispersive Infrarotdetektoren (NDIR) durchgeführt und anschließend der TOC bzw. DOC berechnet.

Bei der sogenannten UV-Persulfatmethode hingegen wird eine erhitzte Probe zunächst mit Säure versetzt, um den anorganischen Kohlenstoff in CO2 umzuwandeln. CO2 wird dann mittels Stickstoffs aus der Probe ausgetrieben. Danach wird der Probe Persulfat hinzugegeben und die Probe mit UV-Licht bestrahlt. Der enthaltene organische Kohlenstoff wird dann in einem beheizten Reaktor in CO2 umgewandelt und mittels Stickstoffs aus der Probe ausgetrieben. Die Bestimmung der Masse des CO2 wird anschließend ebenfalls über ein NDIR durchgeführt, sodass der TOC bzw. DOC berechnet werden kann.

Beide Messverfahren liefern sehr exakte quantitative Ergebnisse. Erkauft wird diese hohe Genauigkeit jedoch durch mehrere aufwändige (Labor-) Arbeitsschritte, was einen hohen Zeit- und Kostenaufwand mit sich bringt.

Alternativ kann der DOC-Anteil bzw. der TOC-Anteil mittels der sogenannten UV-Adsorbanz-Signalmessung (trübungskompensiertes Signal), auch SAK (spektraler Adsorptionskoeffizient) genannt, ermittelt werden. Bei dieser Methode nutzt man die Tatsache, dass zwischen dem SAK-Wert und dem DOC-Anteil eine Korrelation besteht.

Die Messungmethode basiert darauf, dass zahlreiche gelöste organische Kohlenstoffe aromatische Ringe oder Doppelbindungen aufweisen, welche UV-Licht der Wellenlänge 254 nm adsorbieren. Über die Bestimmung des spektraler Adsorptionskoeffizient (SAK) der Probe in diesem Wellenlängenbereich, kann mittels eines einfachen photometrischen bzw. kalorimetrischen Messprinzips kostengünstig und schnell der DOC-Anteil approximiert werden. Nachteilig ist jedoch, dass die Korrelation zwischen dem SAK-Wert und dem DOC-Anteil wasserspezifisch ist, was bedeutet, dass individuell für einen Messstandort jeweils die Korrelation mittels Vergleichstests (sogenannten Jartests) zuvor ermittelt und regelmäßig überprüft werden muss. Auch kann sich die Korrelation jahreszeitlich ändern, was eine aufwändige Umrechnung von SAK-Wert in TOC erfordert.

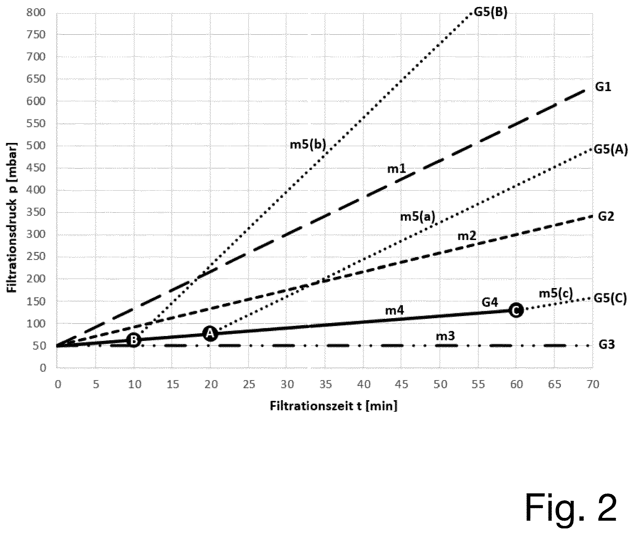

Für zu filterndes Wasser (bzw. Abwasser) ist bekannt, dass ein steigender DOC-Anteil die Leistung des Filters negativ beeinflusst. Grund hierfür ist insbesondere das Anlagern gelöster organischen Kohlenstoffe an der Oberfläche des Filters, das sogenannte organische Fouling:
Die gelösten organischen Kohlenstoffe können einerseits den (Mikro- bzw. Ultrafiltrations)-Filter passieren, andererseits sich aber auch an der Oberfläche des jeweiligen Filters anlagern. Viele gelöste organische Kohlenstoffe, vor allem solche natürlichen Ursprungs wie Huminstoffe, Proteine oder Polysacharide, haben eine hydrophobe (wasserabweisende) Charakteristik. Lagern sich diese an der Oberfläche des Filters an, wird auch der Filter hydrophober (Hydrophobierung des Filters). Dadurch erfährt das Wasser beim Passieren der Filterporen des Filters mehr Widerstand. Bei einem konstanten Flussstrom (Volumenstrom) durch den Filter führt dies zu einem Anstieg des Filtrationsdrucks, was mit einer Reduktion der Filterleistung einhergeht. Das Anlagern von gelösten organischen Kohlenstoffen an der Oberfläche des Filters erfolgt typischerweise kontinuierlich während einer Filtration, womit der Filtrationsdruck auch kontinuierlich über die Filtrationszeit steigt (siehe Fig. 2, Gerade G1 mit Geradensteigung m1) und somit die Filterleistung kontinuierlich sinkt.

Um diesen negativen Einfluss auf die Filterleistung zu minimieren, werden in der Wasser- und Abwasseraufbereitung üblicherweise DOCreduzierende Stoffe (Adsorptionsmittel) vor dem Filter eingesetzt. Hierzu werden metall-basierte Koagulationsmittel, wie z.B. ausfallende Eisenchloride, Aluminiumsulfate, aber auch Pulveraktivkohle (PAK) kontinuierlich vor dem Filter in das zu filternde Medium eindosiert, um den DOC-Anteil (DOC-Konzentration) zu reduzieren. Wenn sich gelöster organischer Kohlenstoff an diesen Adsorptionsmitteln angelagert hat, kann sich dieser nicht mehr an den Filter anlagern und damit die Filterleistung reduzieren. Auf diese Weise kann der Anstieg des Filtrationsdrucks über die Filtrationszeit reduziert werden (siehe Fig. 2, Gerade G2 mit Geradensteigung m2 im Vergleich zur Gerade G1 mit Geradensteigung m1).

Die Veröffentlichung DE 2812 819 A1 beschreibt in diesem Zusammenhang beispielhaft ein Verfahren zur Trinkwasseraufbereitung, bei dem durch Zugabe des Flockungsmittels Eisen-(III)-Chlorid in das aufzubereitende Wasser gelöstes Phosphat ausgefällt und abgeschieden wird.

Die DOC-reduzierenden Stoffe (Adsorptionsmittel) werden bezogen auf Menge und Zumischungszeitpunkt typischerweise derart dosiert, dass die DOC-Aufnahme abgeschlossen ist, bevor die DOC-reduzierenden Stoffe in Kontakt mit dem Filter kommen. Dies wird üblicherweise erreicht, indem die DOC-reduzierenden Stoffe in einem Schnellmixer in das zu filternde Medium (Wasser) eindosiert werden und anschließend in einem Verweilbecken so lange in Schwebe gehalten werden, bis die DOC-Aufnahme abgeschlossen ist und das Wasser erst dann auf den Filter trifft. Zum Zeitpunkt des Kontaktes mit dem Filter sind die DOC-reduzierenden Stoffe idealerweise verbraucht, also bereits mit DOC gesättigt, sodass keine weitere DOC-Aufnahme stattfindet. Die maximale Aufnahmemenge bzw. Adsorptionskapazität wird somit vor dem Erreichen des Filters erzielt, sodass der verbleibende "freie" DOC-Anteil im Wasser reduziert wird. Dadurch ist der Filter vor organischem Fouling besser geschützt.

Während der Filtrationszeit wächst kontinuierlich ein Filterkuchen aus gesättigten DOC-reduzierenden Stoffen (Adsorptionsmittel) an der Filteroberfläche des Filters an. Da die DOC-reduzierenden Stoffe bereits gesättigt sind, findet dabei keine weitere DOC-Aufnahme durch das gesättigte Adsorptionsmittel statt. Damit ist der "freie" DOC Anteil im Wasser, im Filter selbst und im gefilterten Wasser konstant. Die mengenmäßige Dosierung sowie die Sättigungsdauer des Adsorptionsmittels werden üblicherweise zuvor mittels Vergleichstests (Jartests) ermittelt und festgelegt.

Die Veröffentlichung CH707684A2 offenbart in diesem Zusammenhang ein Verfahren zur Wasserreinigung, bei dem der sich an der Filteroberfläche absetzende Filterkuchen ("zurückgehaltenes Adsorbens") durch Rückspülen entfernt und zumindest teilweise einer erneuten Verwendung zugeführt wird.

In der Veröffentlichung DE 10 2014 107 489 A1 wird ein adsorptives Filterverfahren zur Wasseraufbereitung beschrieben, bei dem ein "Adsorptionsmaterial in Form einer kugelförmigen Aktivkohle" mit speziellen Eigenschaften bezüglich Gesamtporenvolumen und Hydrophilie zum Einsatz kommt.

WO2010/088720 offenbart einen Filter, durch den ein zu filtrierendes Medium mit einem DOC-Anteil gepumpt wird. Ein Drucksensor misst den Filtrationsdruck zur Bestimmung der Sättigung des Filters.

Vor diesem Hintergrund liegt der vorliegenden Erfindung die Aufgabe zugrunde ein Verfahren zur Bestimmung der Sättigungsdauer eines Adsorptionsmittels und insbesondere ein Verfahren zum Abschätzen des DOC-Anteils eines zu filtrierenden Mediums mit verbesserter Praxistauglichkeit bereitzustellen, insbesondere in Bezug auf einfache Anwendbarkeit im laufenden Betrieb, exakte Dosierung des Adsorptionsmittels und Prozesseffizienz.

Gelöst wird diese Aufgabe durch das Verfahren zum Bestimmen der Sättigungsdauer eines Adsorptionsmittels gemäß Anspruch 1 bzw. durch das Verfahren zum Abschätzen des Ist-DOC-Anteils gemäß Anspruch 2.

Das Verfahren zum Bestimmen der Sättigungsdauer des Adsorptionsmittels umfasst die folgenden Schritte:
A) Bereitstellen eines mit dem Adsorptionsmittel beschichteten Filters, wobei
   - der Filter eine Filteraußenseite mit einer Filteraußenoberfläche und eine Filterinnenseite aufweist, ein zu filtrierendes Medium mit einem DOC-Anteil an der Filteraußenseite ansteht und das Filtrat über die Filterinnenseite abführbar ist,
   - das Adsorptionsmittel eine Schicht mit einer Adsorptionsmittelmasse auf der Filteraußenseite ausbildet und geeignet ist, gelösten organischen Kohlenstoff aus dem zu filtrierenden Medium (insbesondere Wasser) zu adsorbieren,
B) Bereitstellen einer Pumpe und eines zwischen der Pumpe und dem Filter angeordneten Druckmessgeräts, wobei
   - die Pumpe geeignet ist das zu filtrierende Medium mit einem Pumpvolumenstrom durch den Filter zu pumpen, und
   - das Druckmessgerät geeignet ist den Filtrationsdruck (gemessen gegenüber dem Umgebungsdruck) zu ermitteln,
C) Starten des Pumpens des zu filtrierenden Mediums durch den Filter,
D) Erfassen des zeitlichen Verlaufs des Filtrationsdrucks (während des Pumpens des zu filtrierenden Mediums durch den Filter) und Ableiten der Filtrationsdruckanstiegsrate als Quotient aus Filtrationsdruckanstieg pro Zeiteinheit, und
E) Identifizieren des Zeitpunkts der Sättigung des Adsorptionsmittels (Sättigungszeitpunkt) als den Zeitpunkt einer signifikanten Veränderung der Filtrationsdruckanstiegsrate und Bestimmen einer zugehörigen Sättigungsdauer als Zeitdauer vom Starten des Pumpens bis zum Sättigungszeitpunkt.

Der Filter ist somit mit einem anfänglich (zumindest weitestgehend) ungesättigten Adsorptionsmittel beschichtet, welches beim Durchströmen mit dem zu filtrierenden Medium ungelöste organische Kohlenwasserstoffe adsorbiert. Nach dem Starten des Pumpens lagert sich der ungelöste organische Kohlenwasserstoff (DOC) konstant am Adsorptionsmittel an, bis der Sättigungszeitpunkt des Adsorptionsmittels erreicht ist. Im Idealfall kann das (anfangs ungesättigte) Adsorptionsmittel auf dem Filter bis zum Sättigungszeitpunkt den gesamten DOC-Anteil des zu filtrierenden Mediums vollständig aufnehmen, sodass der Filter auch vollständig vor organischem Fouling geschützt ist. In diesem Idealfall bleibt der Filtrationsdruck über die Filtrationsdauer konstant, die Filtrationsdruckanstiegsrate ist null und es kommt zu keinem Abfall der Filterleistung (vgl. Fig. 2, Gerade G3 mit Geradensteigung m3). In der Praxis kann dieser Idealfall jedoch nur selten erzielt werden. Stattdessen nimmt das anfangs ungesättigte Adsorptionsmittel auf dem Filter bis zum Sättigungszeitpunkt lediglich einen sehr großen Teil der DOC-Beladung des (zu filtrierenden Mediums) auf, sodass das Medium, welches die Filteroberfläche passiert nur einen geringen DOC-Anteil aufweist und der Filter somit nur marginal einem organischem Fouling ausgesetzt ist. Der Filtrationsdruck nimmt dementsprechend nur sehr geringfügig über die Filtrationszeit zu (vgl. Fig.2, Gerade G4), die Filtrationsdruckanstiegsrate ist sehr gering (vgl. Fig.2, Geradensteigung m4) und der Abfall der Filterleistung ist entsprechend klein.

Der Zeitpunkt der Veränderung der Filtrationsdruckanstiegsrate (also der Knickpunkt des zeitlichen Filtrationsdruckverlaufs), entspricht dem Sättigungszeitpunkt des Adsorptionsmittels. Der Sättigungszeitpunkt ist somit jener Zeitpunkt während der Filtration, bei dem die maximale Adsorptionskapazität des Adsorptionsmittels erreicht ist. Das bedeutet, dass nach dem Erreichen des Sättigungszeitpunktes keine (oder nur insignifikant wenig) weitere DOC-Beladung des Mediums durch das Adsorptionsmittel aufgenommen werden kann und damit der im Medium gelöste organische Kohlenstoff die Adsorptionsmittelschicht passiert und folglich auf den Filter trifft und diesen stärker bzw. schneller hydrophobiert. Das führt zu einem stärkeren Anstieg des Filtrationsdrucks über die Filtrationszeit und schlägt sich in einer signifikanten Veränderung der Filtrationsdruckanstiegsrate nieder, da die Filtrationsdruckanstiegsrate nach Erreichen des Sättigungszeitpunkts signifikant größer ist als vor dem Erreichen des Sättigungszeitpunktes.

Dabei liegt eine signifikante Veränderung (Vergrößerung) der Filtrationsdruckanstiegsrate im Sinne der vorliegenden Erfindung vor, wenn die Filtrationsdruckanstiegsrate des aktuellen Zeitintervalls mindestens um 10% größer ist als die Filtrationsdruckanstiegsrate des vorhergehenden Zeitintervalls, wobei es unter Umständen gegebenenfalls zweckmäßig ist Veränderungen erst als signifikant anzusehen, wenn die Filtrationsdruckanstiegsrate des aktuellen Zeitintervalls mindestens um 20%, 50% oder 100% größer ist als die des vorhergehenden Zeitintervalls.

Dabei hat sich herausgestellt, dass diese signifikante Veränderung besonders zuverlässig bestimmt werden kann, wenn das aktuelle Zeitintervall eine Länge von mindestens einer Minute aufweist und das vorherige Zeitintervall der Zeitdauer vom Beginn des Pumpens bis zum Beginn des aktuellen Zeitintervalls entspricht und damit die zugehörige Filtrationsdruckanstiegsrate die durchschnittlichen bisherigen Filtrationsdruckanstiegsrate beschreibt.

Die Ermittlung des Sättigungszeitpunkts bzw. der Sättigungsdauer des Adsorptionsmittels kann somit unmittelbar im laufenden Filterbetrieb stattfinden. Wird mithilfe des erfindungsgemäßen Verfahrens erkannt, dass das Adsorptionsmittel gesättigt ist, kann diese Information unmittelbar für die Steuerung des weiteren Filtrationsprozesses genutzt werden, indem z.B. die Filtration unterbrochen und das Adsorptionsmittel erneuert wird. Auf diese Weise kann die Adsorptionskapazität des Adsorptionsmittels bestmöglich ausgenutzt und Verschwendung minimiert werden.

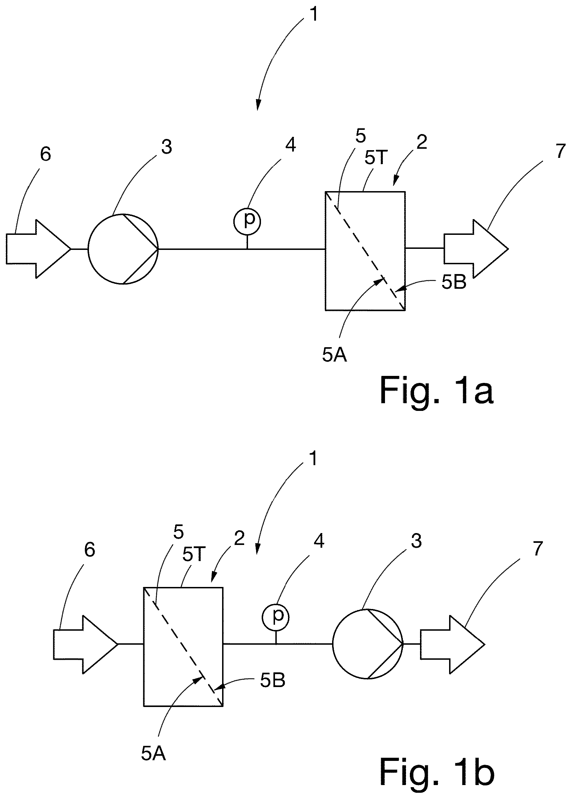

Zur Messung des Filtrationsdrucks können gängige Druckmessgeräte, wie z.B. Drucksensoren oder Druckmanometer verwendet werden. Das Druckmessgerät wird dabei zwischen dem Filter und der Pumpe installiert und misst den Filtrationsdruck gegenüber dem Umgebungsdruck. Bei sogenannten druckbetriebenen Filtern sind die Pumpe und der Filter stromaufwärts vom Filter (also auf der Druckseite des Filters) angeordnet (vgl. Fig. 1a), wohingegen bei saugbetriebenen Filtern die Pumpe und der Filter stromabwärts vom Filter (also auf der Filtratseite des Filters) angeordnet sind (vgl. Fig. 1b). Während der Filtration wird Wasser durch den Filter transportiert und der Filtrationsdruck vor dem Filter kontinuierlich gemessen.

Das erfindungsgemäße Verfahren ermöglicht es, die Sättigungsdauer des Adsorptionsmittels über den Verlauf des Filtrationsdrucks zu bestimmen. Da der Filtrationsdruck einen einfach zu ermittelnden Parameter darstellt, der oftmals bereits aus anderen Beweggründen heraus erfasst wird, gelingt die Bestimmung der Sättigungsdauer auf diese Weise mit minimalem Aufwand.

Ferner ermöglicht das erfindungsgemäße Verfahren einen effizienteren Filterprozess, da darauf verzichtet werden kann, das zu filtrierendes Medium - wie im Stand der Technik üblich - solange in einem Verweilbecken verweilen zu lassen, bis die gelösten organischen Kohlenwasserstoffe durch das Adsorptionsmittel gebunden sind.

Basierend auf dem soeben beschriebenen Verfahren zum Bestimmen der Sättigungsdauer des Adsorptionsmittels umfasst das Verfahren zum Abschätzen des Ist-DOC-Anteils (d.h. zum Ermitteln des ungefähren Ist-DOC-Anteils) eines zu filtrierenden Testmediums die folgenden Schritte:
F) Bestimmen der Ist-Sättigungsdauer des zu filtrierenden Testmediums gemäß dem erfindungsgemäßen Verfahren zum Bestimmen der Sättigungsdauer des Adsorptionsmittels, und
G) Abschätzen des Ist-DOC-Anteils (d.h. Ermitteln des ungefähren Ist-DOC-Anteils) des zu filtrierenden Testmediums durch Division einer Kalibrierkonstante durch die Ist-Sättigungsdauer.

Nachdem für das zu filtrierende Testmedium mit unbekanntem DOC-Anteil (Ist-DOC-Anteil) die Sättigungsdauer als Ist-Sättigungsdauer bestimmt wurde, kann auf diese Weise der Ist-DOC-Anteil abgeschätzt werden (d.h. ungefähr ermittelt werden), indem die Kalibierkonstante durch die ermittelte Ist-Sättigungsdauer dividiert wird.

Die Erfindung basiert auf der Erkenntnis, dass der DOC-Anteil über den korrelierenden Messparameter der Sättigungsdauer direkt ungefähr ermittelt bzw. abgeschätzt werden kann, wobei - bei sonst gleichen Bedingungen - eine kürzere Sättigungsdauer auf einen höheren DOC-Anteil schließen lässt.

Auf diese Weise kann der DOC-Anteil des zu filtrierenden Testmediums unmittelbar im laufenden Filterbetrieb mit nur minimalem Aufwand abgeschätzt und gemonitort werden und eine wertvolle Informationsgrundlage für die weitere Prozessführung liefern. Das Verfahren kann damit vorteilhafterweise auch im sogenannten Hauptstrom eines Filtrationsprozesses angewendet werden, da die erforderliche Filtrationsdruckmessung im Hauptstrom des Filtrationsprozesses in der Regel ohnehin bereits installiert ist.

Gemäß einer bevorzugten Ausführungsform des Verfahrens erfolgt das Bestimmen der Kalibrierkonstante dabei,
- indem für ein Kalibriermedium mit einem bekannten DOC-Anteil (Kalibrier-DOC-Anteil) die Sättigungsdauer (Kalibrier-Sättigungsdauer) gemäß dem oben beschriebenen Verfahren zur Bestimmung der Sättigungsdauer bestimmt wird, und
- die Kalibrierkonstante eine Funktion des Kalibier-DOC-Anteils und der Kalibrier-Sättigungsdauer ist.

Zur Bestimmung der Kalibrierkonstante ist für ein Kalibriermedium, welches möglichst ähnlich zum zu filtrierenden Medium ist, der (Kalibrier-)DOC-Anteil und die (Kalibrier-)Sättigungsdauer zu bestimmen. Als Kalibriermedium wird somit sinnvollerweise das Medium aus jener Quelle verwendet, die später auch das zu filternde Testmedium liefert. Mittels gängiger oben beschriebener Messmethoden (bspw. nach Normung DIN EN1484, ASTM D4839, ASTM D4779) wird dann der DOC-Anteil des Kalibiermediums bestimmt und als Kalibier-DOC-Anteil verwendet und anschließend mittels des oben beschriebenen Verfahrens zur Bestimmung der Sättigungsdauer die Sättigungsdauer für das Kalibiermedium bestimmt.

Die Qualität des zu filternden Mediums (insbesondere Wasser) und damit auch der DOC-Anteil können sich im Laufe der Zeit dauerhaft ändern. Daher muss in regelmäßigen Abständen eine Überprüfung des Kalibrier-DOC-Anteils stattfinden und die zugehörige Kalibrier-Sättigungsdauer neu bestimmt werden. Hierzu kann der Quelle, die das zu filternde Testmedium liefert, in regelmäßig Abständen eine Probe entnommen werden, die dann als Kalibriermedium herangezogen wird.

Dabei erfolgt vorteilhafterweise das Bestimmen der Ist-Sättigungsdauer des zu filtrierenden Testmediums und das Bestimmen der Kalibrier-Sättigungsdauer des Kalibriermediums bei identischen Pumpvolumenströmen, identischen Filteraußenoberflächen und identischen Adsorptionsmittelmengen unter Einsatz des gleichen Adsorptionsmittels.

Denn die Erfinder haben erkannt, dass unter diesen Bedingungen der DOC-Anteil und die Sättigungsdauer näherungsweise indirekt proportional zueinander sind, sodass - gemäß einer weiterhin bevorzugten Ausführungsform des erfindungsgemäßen Verfahrens - die Kalibrierkonstante das Produkt des Kalibier-DOC-Anteils und der Kalibrier-Sättigungsdauer ist.

Die Pumpvolumenströme, die Adsorptionsmittelart, die Adsorptionsmittelmenge und die Filteraußenoberfläche sind somit festgelegte Prozessgrößen. Nur für diese festgelegten Prozessgrößen hat die ermittelte Kalibier-Sättigungsdauer ihre Gültigkeit.

Gemäß einer weiteren bevorzugten Ausführungsform der Erfindung erfolgt das Bereitstellen des mit einem Adsorptionsmittel beschichteten Filters gemäß Schritt A), indem das ungesättigte Adsorptionsmittel mittels eines Anschwemmfiltrationsverfahrens auf die Filteraußenseite aufgetragen wird.

Hierzu wird das ungesättigte Adsorptionsmittel, welches sich auf dem Filter als Beschichtung anlagern soll zunächst vollständig einem Anschwemmmedium zudosiert und mit diesem gemischt. Anschließend wird das Anschwemmmedium mittels der Pumpe durch den Filter gepumpt, sodass sich das ungesättigte Adsorptionsmittel gleichmäßig und homogen an der Filteraußenseite des Filters anlagert und auf der Filteraußenseite eine (Adsorptionsmittel)-Schicht ausbildet. Bevorzugterweise beträgt die Dicke der Adsorptionsmittelschicht dabei zwischen 0,1 und 3 mm.

Als Anschwemmmedium kann dabei das zu filtrierende Medium zum Einsatz kommen. Das Adsorptionsmittel adsorbiert dann, während es an die Filteroberfläche angeschwemmt wird, bereits DOC aus dem zu filtrierenden Medium und ist somit im strengen Sinne nicht mehr vollständig ungesättigt, wenn sich die Adsorptionsmittelschicht auf dem Filter ausgebildet hat. Da dieser Anschwemmvorgang allerdings relativ zügig von statten geht, ist davon auszugehen, dass das Adsorptionsmittel zu dem Zeitpunkt, wenn der Anschwemmvorgang abgeschlossen ist und die Absorptionsmittelschicht ausgebildet ist zumindest weitestgehend ungesättigt ist.

Nach Abschluss der Filtration wird der Filter gemäß einer weiteren bevorzugen Ausführungsform zurückgespült, indem das (dann gesättigte) Adsorptionsmittel von der Filteraußenseite durch Rückspülung abgetrennt wird. Auf diese Weise können die am Filter angelagerten Feststoffe inklusive des Adsorptionsmittels vollständig abgetragen und aus einem Filtrationstank (in dem der Filter angeordnet ist) entfernt werden. Nach der Entfernung erfolgt erneut das Befüllen des Filtrationstanks mit dem Anschwemmmedium und dem Adsorptionsmittel und das Anschwemmen beginnt erneut.

Gemäß einer weiteren bevorzugten Ausführungsform des erfindungsgemäßen Verfahrens ist der Filter als Mikro- bzw. Ultrafiltrationsfilter ausgeführt und weist Filterporen mit einem Filterporendurchmesser von 0,05 bis 2,0 µm auf, und weist das Adsorptionsmittel Partikel mit einer Partikelgröße von 5 bis 500 µm auf. Die Partikelgröße des Adsorptionsmittels ist damit deutlich größer als die Porendurchmesser des Filters. Somit kann erreicht werden, dass sich kein Adsorptionsmittel in die Poren einlagert und die Poren verblockt oder gar durch den Filter durchdringt. Stattdessen bildet sich in vorteilhafter Weise eine lockere Adsorptionsmittelschicht an der Oberfläche des Filters aus.

Vorteilhafterweise ist der Filter als Keramikfilter, Glasfilter, Metallfilter oder Kunststofffilter ausgeführt. Aus all diesem Materialen können Filter mit Porendurchmessern im gewünschten Mikro- bzw. Ultrafiltrationsfilterbereich hergestellt werden.

Das Adsorptionsmittel umfasst vorteilhafterweise Pulveraktivkohle (PAK) oder ein metallbasiertes Koagulationsmittel, insbesondere Eisenchloride oder Aluminiumsulfate.

Nachfolgend werden die erfindungsgemäßen Verfahren anhand der Zeichnung näher erläutert. Dabei zeigt
- Fig. 1a und 1b: jeweils schematisch eine Filtrationsvorrichtung umfassend eine Pumpe, ein Druckmessgerät und eine Filterkonfiguration, wobei in Fig. 1a ein druckbetriebener Filter und in Fig. 1b ein saugbetriebener Filter darstellt ist,
- Fig. 2: schematische Filtrationsdruckverläufe für Filtrationsvorrichtungen gemäß Fig. 1a oder 1b, und
- Fig. 3: gemessene Filtrationsdruckverläufe für eine Filtrationsvorrichtung gemäß Fig. 1b.

Figur 1a und 1b zeigt jeweils schematisch eine Filtrationsvorrichtung 1 umfassend eine Filterkonfiguration 2, eine Pumpe 3 und ein zwischen der Filterkonfiguration 2 und der Pumpe 3 angeordnetes Druckmessgerät 4. Die Filterkonfiguration 2 umfasst einen Filter 5 mit einer Filteraußenseite 5A und einer Filterinnenseite 5B und einen Filtrationstank 5T, in dem der Filter 5 angeordnet ist.

In Figur 1a ist ein druckbetriebener Filter dargestellt. Die Pumpe 3 und die Druckmessvorrichtung 4 sind dabei stromaufwärts von der Filterkonfiguration 2, also auf der der Filteraußenseite 5A zugewandten Seite des Filters 5, angeordnet.

Figur 1b hingegen zeigt einen saugbetriebenen Filter. Hier sind die Pumpe 3 und die Druckmessvorrichtung 4 stromabwärts von der Filterkonfiguration 2, also auf der der Filterinnenseite 5B angeordneten Seite des Filters 5, angeordnet.

Die Pumpe 3 ist jeweils geeignet ein zu filtrierendes Medium (insbesondere Wasser) von der Quelle 6 durch den Filter (also von der Filteraußenseite 5A zur Filterinnenseite 5B) zur Senke 7 zu pumpen (bzw. zu saugen).

Beim druckbetriebenen Filter gemäß Fig. 1a ermittelt die Druckmessvorrichtung 4 den Filtrationsdruck p (gegenüber dem Umgebungsdruck), der sich im zu filtrierenden Medium stromaufwärts vor der Filterkonfiguration 2 einstellt. Beim saugbetriebenen Filter gemäß Fig. 1b ermittelt die Druckmessvorrichtung 4 den Filtrationsdruck p (gegenüber dem Umgebungsdruck), der sich im Filtrat stromabwärts von der Filterkonfiguration 2 einstellt.

In Figur 2 sind mehrere schematische Verläufe des Filtrationsdrucks p in mbar über die Filtrationszeit t in Minuten (min) für Filtrationsvorrichtungen 1 gemäß der Figur 1a oder 1b dargestellt.

Die Gerade G1 stellt dabei den schematischen Verlauf des Filtrationsdrucks p über die Filtrationszeit t dar, wenn ein zu filtrierendes Medium mit einem gewissen DOC-Anteil DOC_1 durch den Filter 5 der Filtervorrichtung 2 gepumpt wird und keinerlei Maßnahmen zur Adsorption des DOC ergriffen werden. Gelöster organischer Kohlenstoff lagert sich auf der Filteraußenseite 5A des Filters 5 ab und führt zu organischem Fouling. Dem durch den Filter 5 strömenden zu filterndem Medium (Wasser) wird durch die fortschreitende Hydrophobierung des Filters 5 ein immer größerer Widerstand entgegengesetzt, was sich in einem kontinuierlich steigenden Filtrationsdruck p niederschlagt. Die Filtrationsdruckanstiegsrate stellt den Quotienten aus dem Filtrationsdruckanstieg pro Zeiteinheit dar und entspricht graphisch der Geradensteigung der jeweiligen Geraden.

Der durch die Gerade G2 schematisch dargestellte Filtrationsdruckverlauf ergibt sich, wenn dem zu filtrierenden Medium zunächst ein Adsorptionsmittel beigemischt wird und das Medium erst dann auf den Filter 5 trifft, nachdem der DOC-Anteil des Mediums durch das Adsorptionsmittel reduziert wurde. Auch hier lagert sich mit zunehmender Filtrationszeit t gelöster organischer Kohlenstoff an der Filteroberfläche (bzw. auf der Filteraußenseite 5A) ab, führt zu organischem Fouling und einem kontinuierlich ansteigenden Filtrationsdruck p. Die Filtrationsdruckanstiegsrate m2 fällt bei G2 allerdings geringer aus als bei G1, weil der DOC-Anteil des auf den Filter treffenden Mediums geringer ist. Der Filterleistungsverlust fällt somit geringer aus.

Die Geraden G3, G4 sowie G5(A), G5(B) und G5(C) hingegen illustrieren schematisch Filtrationsdruckverläufe, die sich bei Anwendung der erfindungsgemäßen Verfahren einstellen. Zu Beginn der Filtrationszeit t (also bei t=0) wird die Pumpe gestartet, um das zu filtrierende Medium durch den Filter zu pumpen und ist der Filter 5 der Filtrationsvorrichtung 2 mit einem ungesättigten Adsorptionsmittel beschichtet. Das ungesättigte Adsorptionsmittel bildet dabei eine (Adsorptionsmittel-)Schicht mit einer Adsorptionsmittelmasse auf der Filteraußenseite 5A aus und ist geeignet gelösten organischen Kohlenstoff aus dem zu filtrierenden Medium zu adsorbieren.

Der idealtypische Verlauf gemäß Gerade G3 ergibt sich für den Fall, wenn die Adsorptionsmittelschicht den DOC-Anteil vollständig aus dem zu filtrierenden Medium herausfiltert. Das auf die Filteraußenseite 5A des Filters 5 treffende Medium ist demnach vollständig von DOC befreit, sodass organisches Fouling vollständig unterbunden wird. Der Filtrationsdruck p bleibt konstant, die Filtrationsdruckanstiegsrate m3 ist somit Null und Filtrationsleistungsverluste können (bis zum Sättigungszeitpunkt) vollständig verhindert werden (Innerhalb der in Fig. 2 dargestellten Filtrationszeit von 70 min wird der Sättigungszeitpunkt des Adsorptionsmittels nicht erreicht). Dieser Idealfall wird in der Praxis allerdings nur sehr selten anzutreffen sein.

Eher wird sich in der Praxis der durch die Geraden G4 und G5(A), G5(B) bzw. G5(C) skizzierte Fall einstellen. Dabei adsorbiert die Adsorptionsmittelschicht zwar einen Großteil des DOC-Anteils des zu filtrierenden Mediums - aber eben nicht alles. Der nicht-adsorbierte Teil des DOC-Anteils trifft somit auf die Filteraußenseite 5A, lagert sich dort (zumindest teilweise) ab und verursacht organisches Fouling, was sich in einer Hydrophobierung des Filters und damit in einem kontinuierlichen Anstieg des Filtrationsdrucks p mit der Filtrationsdruckanstiegsrate m4 niederschlägt. Nach einer gewissen Filtrationszeit t ist die Adsorptionskapazität der Adsorptionsmittelschicht ausgeschöpft und dem zu filtrierenden Medium kann kein weiteres DOC mehr entzogen werden. Dieser Zeitpunkt (in Fig. 2 als A, B, C illustriert) wird auch als Sättigungszeitpunkt bezeichnet. Folglich kommt nach dem Sättigungszeitpunkt pro Zeiteinheit mehr DOC an der Filteraußenseite an und lagert sich dort ab, wodurch der Anstieg des Filtrationsdrucks p beschleunigt wird. Dies führt dazu, dass die Filtrationsdruckanstiegsrate m5(a), m5(b), m5(c) nach dem jeweiligen Sättigungszeitpunkt größer ist als vor dem Sättigungszeitpunkt, sodass der Sättigungszeitpunkt als Knick des Filtrationsdruckverlaufs erscheint.

Für ein zu filtrierendes Medium mit einem ersten DOC-Anteil DOC_A ergibt sich damit ein Sättigungszeitpunkt A bei der Filtrationszeit t = 20 min, die Sättigungsdauer des Adsorptionsmittels beträgt demnach 20 min. Für einen DOC-Anteil DOC_B, der doppelt so groß ist wie der DOC-Anteil DOC_A, halbiert sich die Sättigungsdauer - bei sonst gleichen Bedingungen - auf 10 min (siehe Sättigungszeitpunkt B). Für einen DOC-Anteil DOC_C, der nur ein Drittel des DOC-Anteils DOC_A beträgt, verdreifacht sich die Sättigungsdauer auf 60 min (siehe Sättigungszeitpunkt C).

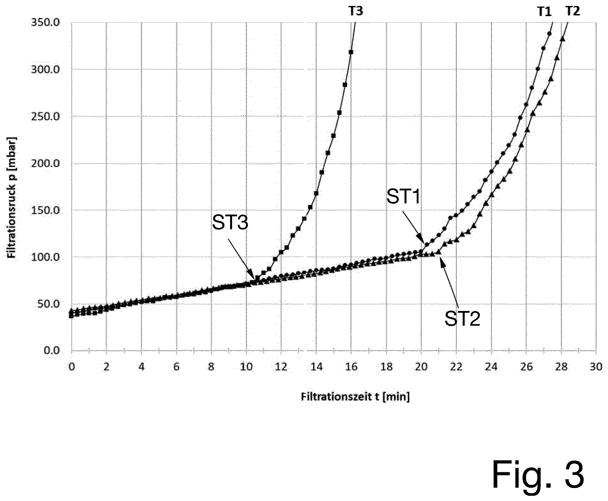

Figur 3 hingegen stellt im Gegensatz zu Figur 2 keine schematischen, sondern drei real gemessene Filtrationsdruckverläufe (Messungen) T1 bis T3 dar, die sich bei Anwendung der erfindungsgemäßen Verfahren in einer Filtrationsvorrichtung gemäß Figur 1b eingestellt haben. Allen dargestellten Filtrationsdruckverläufen T1 bis T3 ist gemein, dass der Pumpvolumenstrom 250 Liter pro Stunde und 1 m² Filteraußenoberfläche betrug und als Adsorptionsmittel Pulveraktivkohle zum Einsatz kam, die anfangs (also zur Filtrationszeit t=0) eine Adsorptionsmittelschicht von 10 g/m² Filteraußenoberfläche ausbildete.

Für die Filtrationsdruckverläufe T1 und T2 wurde als zu filtrierendes Medium Flusswasser mit einem DOC-Anteil von 8,1 mg/l, für den Filtrationsdruckverlauf T3 ein Flusswasser mit einem DOC-Anteil von 15,2 mg/l verwendet. Etwa dreimal pro Minute wurde nach dem Starten der Pumpe der gemessene Filtrationsdruck p ermittelt und gegenüber der verstrichenen Filtrationszeit aufgetragen. Es zeigt sich, dass die Filtrationsdruckverläufe T1 bis T3 zunächst mit einer nahezu konstanten Filtrationsdruckanstiegsrate ansteigen, bis die jeweiligen Sättigungszeitpunkte ST1, ST2 bzw. ST3 erreicht werden und sich die Filtrationsdruckanstiegsraten signifikant verändern bzw. vergrößern. Die Sättigungszeitpunkte ST1 und ST2 befinden sich bei einer Filtrationszeit von ca. 20,5 min bzw. 21 min. Der Unterschied zwischen den Filtrationsdruckverläufen T1 und T2 ist größtenteils Messungenauigkeiten zuzuschreiben. Der Sättigungszeitpunkt ST3 befindet sich bei einer Filtrationszeit von etwa 10,5 min. Damit ist die zugehörige Sättigungsdauer des Filtrationsdruckverlaufs T3 etwa halb so groß wie bei den Filtrationsdruckverläufen T1 und T2, während der DOC-Anteil des Filtrationsdruckverlaufs T3 doppelt so groß ist wie bei T1 und T2. Die Messwerte bestätigen damit die Erkenntnis, dass die Sättigungsdauer - bei sonst gleichbleibenden Bedingungen - indirekt proportional zum DOC-Anteil ist.

In allen drei Messungen T1 bis T3 lag der DOC-Anteil im Filtrat (also dem gefilterten Wasser) vor Erreichen des Sättigungszeitpunktes im Bereich von 0,94 - 1,11 mg/l. Damit ergibt sich eine Adsorptionsrate von 7,16 bzw. 7,07 mg/l für die Messungen T1 bzw. T2 und für die Messung T3 eine Adsorptionsrate von 14,09 mg/l. In der Messung T3 nimmt also das Adsorptionsmittel infolge des annähernd doppelt so hohen DOC-Anteils des zu filtrierenden Mediums (Flusswasser) auch doppelt so viel an DOC auf. Als Ergebnis halbiert sich die Filtrationszeit bis zum Sättigungszeitpunkt von 20,5 bzw. 21,0 min bei Messung T1 bzw. T2 auf nur 10,5 min bei der Messung T3. Nach Erreichen des Sättigungszeitpunktes des Adsorptionsmittels näherten sich der DOC-Anteil im Filtrat (gefiltertes Wasser) dem DOC-Anteil des zu filtrierenden Mediums (Flusswasser) an. Die DOC-Anteile im Filtrat (gefiltertes Wasser) lagen im Bereich von 7,61 - 7,33 mg/l für die Messungen T1 und T2, für die Messung T3 betrug der entsprechende Wert 14,14 mg/l.

Der Filter selbst tritt nach dem Sättigungszeitpunkt mit Flusswasser mit höheren DOC-Anteilen in direkten Kontakt. Dadurch kann sich nun mehr und schneller DOC als (organisches) Fouling an der Oberfläche des Filters anlagern, wodurch im Ergebnis der Filtrationsdruck schneller ansteigt. Gut zu erkennen ist ebenfalls, dass infolge eines etwa doppelt so hohen DOC-Anteils im Filtrat (gefilterten Wasser) der Messung T3 auch die zugehörige Filtrationsdruckanstiegsrate nach dem Sättigungszeitpunkt etwa doppelt so groß ist wie bei den Messungen T1 und T2.

## Patentansprüche

1. Verfahren zur Bestimmung der Sättigungsdauer eines Adsorptionsmittels mit den folgenden Schritten:
A) Bereitstellen eines mit dem Adsorptionsmittel beschichteten Filters (5), wobei
- der Filter (5) eine Filteraußenseite (5A) mit einer Filteraußenoberfläche und eine Filterinnenseite (5B) aufweist, ein zu filtrierendes Medium mit einem DOC-Anteil an der Filteraußenseite (5A) ansteht und das Filtrat über die Filterinnenseite (5B) abführbar ist,
- das Adsorptionsmittel eine Schicht mit einer Adsorptionsmittelmasse auf der Filteraußenseite (5A) ausbildet und geeignet ist, gelösten organischen Kohlenstoff aus dem zu filtrierenden Medium zu adsorbieren,
B) Bereitstellen einer Pumpe (3) und eines zwischen der Pumpe (3) und dem Filter (5) angeordneten Druckmessgeräts (4), wobei
- die Pumpe (3) geeignet ist das zu filtrierende Medium mit einem Pumpvolumenstrom durch den Filter (5) zu pumpen, und
- das Druckmessgeräts (4) geeignet ist den Filtrationsdruck (p) zu ermitteln,
C) Starten des Pumpens des zu filtrierenden Mediums durch den Filter (5),
D) Erfassen des zeitlichen Verlaufs des Filtrationsdrucks (p) und Ableiten der Filtrationsdruckanstiegsrate als Quotient aus Filtrationsdruckanstieg pro Zeiteinheit, und
E) Identifizieren des Sättigungszeitpunkt (A, B, C, ST1, ST2, ST3) des Adsorptionsmittels als den Zeitpunkt einer signifikanten Veränderung der Filtrationsdruckanstiegsrate und Bestimmen einer zugehörigen Sättigungsdauer als Zeitdauer vom Starten des Pumpens bis zum Sättigungszeitpunkt.

2. Verfahren zum Abschätzen des Ist-DOC-Anteils eines zu filtrierenden Testmediums mit den folgenden Schritten:
F) Bestimmen der Ist-Sättigungsdauer des zu filtrierenden Testmediums gemäß Anspruch 1, und
G) Ermitteln des ungefähren Ist-DOC-Anteils des zu filtrierenden Testmediums durch Division einer Kalibrierkonstante durch die Ist-Sättigungsdauer.

3. Verfahren nach Anspruch 2, wobei das Bestimmen der Kalibrierkonstante erfolgt,
- indem für ein Kalibriermedium mit einem bekannten DOC-Anteil (Kalibrier-DOC-Anteil) die Sättigungsdauer (Kalibrier-Sättigungsdauer) gemäß Anspruch 1 bestimmt wird, und
- die Kalibrierkonstante eine Funktion des Kalibier-DOC-Anteils und der Kalibrier-Sättigungsdauer ist.

4. Verfahren nach Anspruch 3, wobei das Bestimmen der Ist-Sättigungsdauer des zu filtrierenden Testmediums und das Bestimmen der Kalibrier-Sättigungsdauer des Kalibriermediums bei identischen Pumpvolumenströmen, identischen Filteraußenoberflächen und identischen Adsorptionsmittelmengen unter Einsatz des gleichen Adsorptionsmittels erfolgt.

5. Verfahren nach Anspruch 4, wobei die Kalibrierkonstante das Produkt des Kalibier-DOC-Anteils und der Kalibrier-Sättigungsdauer ist.

6. Verfahren nach einem der vorstehenden Ansprüche, wobei das Bereitstellen des mit einem Adsorptionsmittel beschichteten Filters (5) gemäß Schritt A) erfolgt, indem das ungesättigte Adsorptionsmittel mittels eines Anschwemmfiltrationsverfahrens auf die Filteraußenseite (5A) aufgetragen wird.

7. Verfahren nach einem der vorstehenden Ansprüche mit dem zusätzlichen Schritt:
H) Abtrennen des Adsorptionsmittels von der Filteraußenseite (5A) durch Rückspülung.

8. Verfahren nach einem der vorstehenden Ansprüche, wobei
- der Filter (5) Filterporen mit einen Filterporendurchmesser von 0,05 bis 2,0 µm aufweist, und
- das Adsorptionsmittel Partikel mit einer Partikelgröße von 5 bis 500 µm aufweist.

9. Verfahren nach einem der vorstehenden Ansprüche, wobei der Filter (5) als Keramikfilter, Glasfilter, Metallfilter oder Kunststofffilter ausgeführt ist.

10. Verfahren nach einem der vorstehenden Ansprüche, wobei das Adsorptionsmittel Pulveraktivkohle oder ein metallbasiertes Koagulationsmittel, insbesondere Eisenchloride oder Aluminiumsulfate, umfasst.

## Claims

1. A method for determining the saturation duration of an adsorbent comprising the following steps:
A) providing a filter (5) coated with the adsorbent, wherein
- the filter (5) has a filter outer side (5A) with a filter outer surface and a filter inner side (5B), a medium to be filtered with a DOC content is present on the filter outer side (5A) and the filtrate can be discharged via the filter inner side (5B),
- the adsorbent forms a layer with an adsorbent mass on the outside of the filter (5A) and is suitable for adsorbing dissolved organic carbon from the medium to be filtered,
B) providing a pump (3) and a pressure measuring device (4) arranged between the pump (3) and the filter (5), wherein
- the pump (3) is suitable for pumping the medium to be filtered through the filter (5) at a pump flow rate, and
- the pressure measuring device (4) is suitable for determining the filtration pressure (p),
C) starting of pumping the medium to be filtered through the filter (5),
D) recording the temporal course of the filtration pressure (p) and deriving the filtration pressure increase rate as a quotient of filtration pressure increase per time unit, and
E) identifying the saturation time (A, B, C, ST1, ST2, ST3) of the adsorbent as the time of a significant change in the filtration pressure increase rate and determine an associated saturation duration as the time from the start of pumping to the saturation time.

2. Method for estimating the actual DOC content of a test medium to be filtered, comprising the following steps:
F) determining the actual saturation time of the test medium to be filtered according to claim 1, and
G) determining the approximate actual DOC content of the test medium to be filtered by dividing a calibration constant by the actual saturation duration.

3. The method according to claim 2, wherein the calibration constant is determined,
- by determining the saturation duration (calibration saturation duration) for a calibration medium with a known DOC content (calibration DOC content) according to claim 1, and
- the calibration constant is a function of the calibration DOC content and the calibration saturation duration.

4. The method according to claim 3, wherein the determination of the actual saturation duration of the test medium to be filtered and the determination of the calibration saturation duration of the calibration medium are carried out at identical pump volume flows, identical filter outer surfaces and identical adsorbent quantities using the same adsorbent.

5. The method according to claim 4, wherein the calibration constant is the product of the calibration DOC content and the calibration saturation duration.

6. Method according to one of the preceding claims, wherein the filter (5) coated with an adsorbent is provided according to step A) by applying the unsaturated adsorbent to the outside of the filter (5A) by means of a precoat filtration process.

7. Method according to any one of the preceding claims with the additional step:
H) separating the adsorbent from the outside of the filter (5A) by backwashing.

8. Method according to one of the preceding claims, wherein
- the filter (5) has filter pores with a filter pore diameter of 0.05 to 2.0 µm, and
- the adsorbent has particles with a particle size of 5 to 500 µm.

9. Method according to one of the preceding claims, wherein the filter (5) is designed as a ceramic filter, glass filter, metal filter or plastic filter.

10. A process according to any one of the preceding claims, wherein the adsorbent comprises powdered activated carbon or a metal-based coagulant, in particular ferric chlorides or aluminium sulphates.

## Revendications

1. Procédé destiné à déterminer le délai de saturation d'un agent d'adsorption, comportant les étapes suivantes :
A) mise à disposition d'un filtre (5) revêtu avec ledit agent d'adsorption,
- ledit filtre (5) présentant une face extérieure de filtre (5A) pourvue d'une surface extérieure de filtre et une face intérieure de filtre (5B), un milieu à filtrer avec une proportion de COD étant présent sur la face extérieure de filtre (5A), et le filtrat pouvant être évacué à travers la face intérieure de filtre (5B),
- l'agent d'adsorption formant une couche comportant une masse d'agent d'adsorption sur la face extérieure de filtre (5A) et étant approprié à adsorber du carbone organique dissous qui est présent dans le milieu à filtrer,
B) mise à disposition d'une pompe (3) et d'un manomètre (4) disposé entre la pompe (3) et le filtre (5),
- la pompe (3) étant appropriée à pomper le milieu à filtrer à travers le filtre (5) en établissant un flux volumique de pompage, et
- le manomètre (4) étant approprié à déterminer la pression de filtrage (p),
C) démarrage du pompage, à travers le filtre (5), du milieu à filtrer,
D) détection de l'évolution dans le temps de la pression de filtrage (p) et déduction du taux d'augmentation de la pression de filtrage en établissant un quotient exprimant l'augmentation de la pression de filtrage par unité de temps, et
E) identification de l'instant de saturation (A, B, C, ST1, ST2, ST3) de l'agent d'adsorption de manière a ce qu'il corresponde à l'instant où le taux d'augmentation de la pression de filtrage subit un changement significatif, et détermination d'un délai de saturation associé qui correspond au temps qui s'est écoulé entre le début du pompage et ledit instant de saturation.

2. Procédé destiné à estimer la proportion réel de COD d'un milieu d'essai à filtrer, comportant les étapes suivantes :
F) détermination du délai de saturation réel du milieu d'essai à filtrer selon la revendication 1, et
G) établissement de la proportion réel de COD approximative du milieu d'essai à filtrer en divisant une constante de calibrage par le délai de saturation réel.

3. Procédé selon la revendication 2, la constante de calibrage étant déterminée
- en déterminant, pour un milieu de calibrage ayant une proportion de COD connue (proportion de COD de calibrage), le délai de saturation (délai de saturation de calibrage) selon la revendication 1, et
- la constante de calibrage étant une fonction de la proportion de COD de calibrage et du délai de saturation de calibrage.

4. Procédé selon la revendication 3, la détermination du délai de saturation réel du milieu d'essai à filtrer et la détermination du délai de saturation de calibrage du milieu de calibrage étant réalisées avec des flux volumiques de pompage identiques, des surfaces extérieures de filtre identiques et des quantités d'agent d'adsorption identiques, en mettant en œuvre le même agent d'adsorption.

5. Procédé selon la revendication 4, la constante de calibrage correspondant au produit de la proportion de COD de calibrage et du délai de saturation de calibrage.

6. Procédé selon l'une des revendications précédentes, la mise à disposition du filtre (5) revêtu avec un agent d'adsorption selon l'étape A) étant réalisée en appliquant l'agent d'adsorption non-saturé sur la face extérieure de filtre (5A) au moyen du processus de filtrage avec constitution d'une pré-couche.

7. Procédé selon l'une des revendications précédentes, comportant l'étape supplémentaire suivante :
H) séparation de l'agent d'adsorption de la face extérieure de filtre (5A) par rétrolavage.

8. Procédé selon l'une des revendications précédentes,
- ledit filtre (5) comportant des pores filtrants dont le diamètre de pore filtrant est compris entre 0,05 et 2,0 µm, et
- ledit agent d'adsorption comportant des particules dont la taille de particule est comprise entre 5 et 500 µm.

9. Procédé selon l'une des revendications précédentes, le filtre (5) étant réalisé sous forme d'un filtre céramique, filtre en verre, filtre métallique ou de filtre en matière plastique.

10. Procédé selon l'une des revendications précédentes, ledit agent d'adsorption comprenant du charbon actif en poudre ou un agent de coagulation à base de métaux, s'agissant plus particulièrement de chlorures de fer ou de sulfates d'aluminium.
